# EUROPÄISCHE PATENTSCHRIFT

(11) **EP 2 788 062 B1**
(45) Veröffentlichungstag und Bekanntmachung des Hinweises auf die Patenterteilung: **26.09.2018**
(21) Anmeldenummer: 12816445.6
(22) Anmeldetag: 29.11.2012
(51) Int. Cl.: A61M 16/04

(54) **ANORDNUNG IN FORM EINER TRACHEOSTOMA- PROTHESE ZUM LEICHTEN EINSETZEN UND LEICHTEN ENTNEHMEN EINER INNENKANÜLE**
ARRANGEMENT IN THE FORM OF A TRACHEOSTOMA PROSTHESIS FOR EASY INSERTION AND EASY REMOVAL OF AN INNER CANNULA
SYSTÈME SE PRÉSENTANT SOUS LA FORME D'UNE PROTHÈSE DE TRACHÉOSTOME ET DESTINÉ À FACILITER L'INTRODUCTION ET LE RETRAIT D'UNE CANULE INTERNE

(30) Priorität: 05.12.2011 DE 102011120694
(43) Veröffentlichungstag der Anmeldung: 15.10.2014
(73) Patentinhaber: PRIMED HALBERSTADT MEDIZINTECHNIK GMBH, 38820 Halberstadt (DE)
(72) Erfinder: TRÄGER, Peter, 38820 Halberstadt (DE); KLIMENTA, Klaus, 38835 Zilly (DE)
(74) Vertreter: Donath, Dirk
(86) Internationale Anmeldenummer: PCT/DE2012/001182
(87) Internationale Veröffentlichungsnummer: WO 2013/083117

(56) Entgegenhaltungen:
- DE-A1- 3 819 237
- DE-U1-202004 020 109
- GB-A- 634 978
- US-A1- 2008 072 911

## Beschreibung

Die Erfindung betrifft eine Anordnung in Form einer Tracheostoma-Prothese zum leichten Einsetzen und leichten Entnehmen einer Innenkanüle.

Tracheostoma- Prothesen (auch Tracheostomiekanülen, Trachealkanülen, Endotrachealtuben oder Tracheostoma-Tuben genannt) zur Behandlung tracheotomierter bzw. kehlkopfloser (laryngektomierter) Patienten mit eröffneter Kehle (s.g. Tracheostoma) sind seit Jahrzehnten bekannt.

Aus der Schrift DE 195 14 433 A1 ist eine Tracheostomiekanüle zum Einsatz in ein Tracheostoma bekannt, welche aus einer schlauchförmigen Außenkanüle mit Kanülenschild und einer schlauchförmigen Innenkanüle besteht, wobei die Innenkanüle in die Außenkanüle einführbar und mit dieser am proximalen Teil verriegelbar ist.

Der Nachteil dieser Verriegelung ist, dass diese durch Verklemmung schwer lösbar sein kann, was die Entnahme der Innenkanüle aus der Außenkanüle erheblich erschwert.

Aus der P 38 19 237 A1 ein Endotrachealtubus mit einem Außenrohr und einem Innenrohr bekannt, wobei das Innenrohr ein Verriegelungselement für eine Rastverbindung mit dem Außenrohr aufweist. Das Innenrohr besitzt dabei eine mit dem Verriegelungselement verbundene Betätigungshandhabe, welche leicht zugänglich ist und an welcher das Innenrohr aus der Verbindung mit dem Außenrohr herausziehbar ist.

Der Nachteil dieser Schlaufen- bzw. Laschen- förmigen Betätigungshandhabe besteht darin, dass diese bei zu hohen Zugkräften beim Entfernen des Innen- aus dem Außenrohr (bspw. bei einem im Außenrohr verkemmten oder durch Körpersekrete verklebtes Innenrohr bzw. bei unsachgemäßer Handhabung) vom Innenrohr abreisst, so dass in Folge dieses Abreißens das Innenrohr nur noch unter sehr großem Aufwand aus dem Außenrohr entfernt werden kann.

Durch das Geschmacksmuster 40208362-0001 und dessen Beschreibung ist ein Knüpfverschluss für eine Trachealkanüle bestehend aus einem sechszahnigen, runden Kunststoffteil an der Innenkanüle und einem runden, wulstförmigen Ansatz an der Außenkanüle bekannt. Die Innenkanüle wird durch Abhebeln des Knüpfverschlusses durch seitliches Ansetzen und Hebeln mit Daumen und Zeigefinger am Knüpfverschluss aus der Außenkanüle gelöst. Zur Befestigung der Innenkanüle in der Außenkanüle wird die Innenkanüle nach dem Einführen in die Außenkanüle mit einem leichten Druck auf den Knüpfverschluss gedrückt. Beim Einrasten kommt es zu einem hörbaren Klicken.

Der Nachteil dieses Knüpfverschlusses besteht darin, dass das Entnehmen der Innenkanüle durch Abhebeln des Knüpfverschlusses erfolgen muss, was durch seitliches Ansetzen und Hebeln mit Daumen und Zeigefinger erfolgt. Durch dieses Ansetzen und Hebeln können leicht die Fingernägel von Daumen und Zeigefinger verletzt werden, was die Handhabung dieses Knüpfverschlusses sehr unangenehm gestalten kann.

Die DE 202004 020 109 offenbart ein Tracheostomarohr mit Adapter für die Laryngektomie bestehend aus einem Tracheostomarohr, das an seinem distalen Ende einen Adapter trägt, wobei der Adapter an seinem freien Ende eine Ausnehmung bzw. zwei gegenüberliegende Ausnehmungen in seinem Rand aufweist. Der Adapter dient dem äußeren Hinzufügen von Zubehörteilen (wie bspw. Trachinaze) an ein Tracheostomarohr, in dem diese Zubehörteile formschlüssig an das Tracheostomarohr angekoppelt werden. Dabei sind die Zubehörteile sehr einfach per Eingreifen eines Fingers einer Hand in die Ausnehmung bzw. von Daumen und Zeigefinger einer Hand in die beiden Ausnehmungen wieder von dem Adapter trennbar.
Der Nachteil dieser technischen Lösung besteht darin, dass keine Innenkanüle in die Außenkanüle einführbar ist.

US2008/0072911 offenbart eine Trachealkanüle mit einer Innenkanüle, wobei die Innenkanüle in eine Öffnung am proximalen Teil der Außenkanüle einführbar ist, und an ihrem proximalen Ende einen wulstartigen Rand aufweist.

Die Aufgabe der Erfindung besteht darin, eine Anordnung in Form einer Tracheostoma- Prothese zum leichten Einsetzen und leichten Entnehmen einer Innenkanüle anzugeben, welche die zuvor stehend genannten Nachteile des Standes der Technik vermeidet und insbesondere ermöglicht, dass die Innenkanüle aus einer arretierten Verbindung mit der Außenkanüle aufwandgering mit Daumen und Zeigefinger auch unter Aufwendung größerer Zugkräfte herausziehbar ist.
Gelöst wird diese Aufgabe durch eine Anordnung in Form einer Tracheostoma- Prothese gemäß dem 1. Patentanspruch. Vorteilhafte Ausgestaltungen der Erfindung sind in den nachgeordneten Ansprüchen angegeben.
Das Wesen der Erfindung besteht darin, dass die Anordnung in Form einer Tracheostoma- Prothese aus einer Außenkanüle und einer in diese Außenkanüle einführbaren Innenkanüle besteht, wobei der äußere Rand des proximalen Teils der Außenkanüle zwei sich gegenüberliegende, kreisbogenförmig ausgeformte Ausnehmungen (so genannte Eingreifmulden) aufweist, welche für Daumen und Zeigefinger eingreifbar ausgestaltet sind, und der äußere Rand des proximalen Teils der Innenkanüle einen wulstartigen Rand aufweist.
Die Erfindung wird nachfolgend an Hand der Figuren näher erläutert. Dabei zeigen:
- Fig. 1:: eine schematische Übersichtsdarstellung einer Ausführungsform einer Außenkanüle der erfindungsgemäßen Anordnung und
- Fig. 2:: eine seitliche Darstellung einer Ausführungsform der erfindungsgemäßen Anordnung mit einer Außen- und einer Innenkanüle.

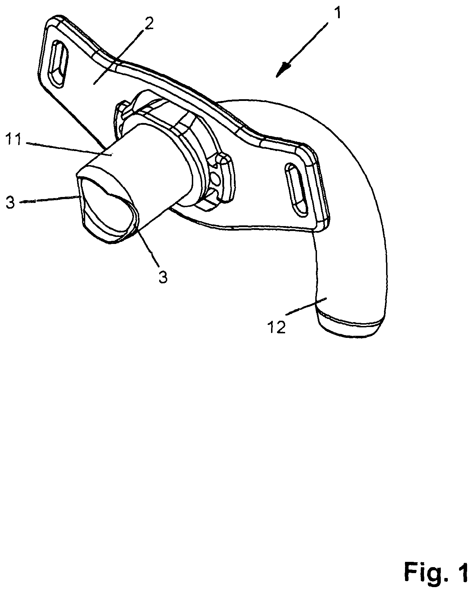

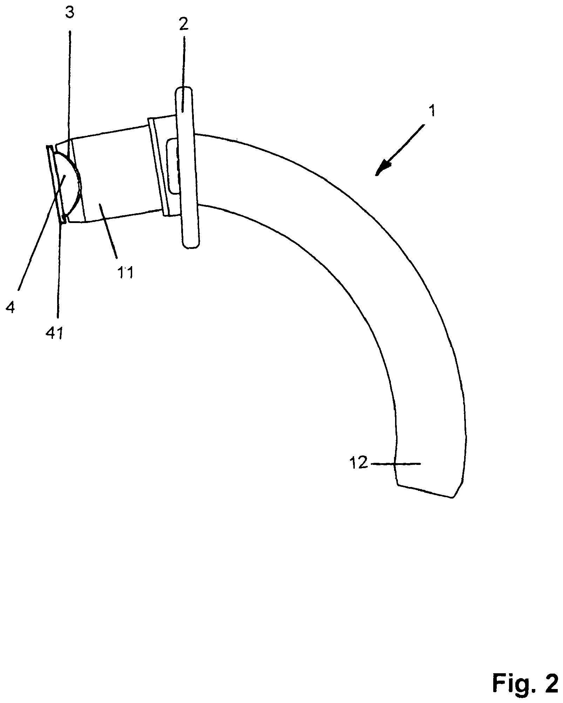

Die in den Figuren 1 und 2 dargestellte Außenkanüle (1) ist gemäß dem Stand der Technik schlauchförmig gestaltet und weist einen proximalen Teil (11) sowie einen distalen Teil (12) auf.

Die in Figur 2 dargestellte, ebenfalls schlauchförmige Innenkanüle (4) weist an ihrem proximalen Ende einen äußere Rand (41) auf, welcher wulstartigen ausgestaltet ist.

In die Öffnung am proximalen Teil (11) der Außenkanüle (1) ist die Innenkanüle (4) einführbar, welche durch geeignete Rast- oder Haltemittel (in der Figur 2 nicht dargestellt) gemäß dem Stand der Technik im proximalen Teil (11) der schlauchförmigen Außenkanüle (1) arretierbar ist.

Die schlauchförmige Außenkanüle (1) kann dabei am proximalen Teil (11) mit einem Kanülenschild (2) gemäß dem Stand der Technik versehen sein.

Erfindungswesentlich ist, dass der äußere Rand des proximalen Teils (11) der Außenkanüle (1) zwei sich gegenüberliegende, kreisbogenförmig ausgeformte Ausnehmungen (3), so genannte Eingreifmulden, aufweist, welche für Daumen und Zeigefinger eingreifbar ausgestaltet sind und dass die Innenkanüle (4) an ihrem proximalen Ende einen äußere Rand (41) auf weist, welcher wulstartigen ausgestaltet ist.

Der Vorteil dieser Ausnehmungen (3) und des wulstartigen äußeren Randes (41) besteht darin, dass eine in der Außenkanüle (1) gemäß dem Stand der Technik arretierte Innenkanüle (4) problemlos und leicht per direktem Zugriff von Daumen und Zeigefinger an dem wulstartigen Rand (41) des im Bereich der Ausnehmungen (3) freiliegenden proximalen Endes der Innenkanüle (4) aus der Außenkanüle (1) entnehmbar ist, und das auch bei stark wirkenden Zugkräften. Die Fingernägel von Daumen und Zeigefinger werden dabei nicht verletzt und es werden dabei auch keine Bestandteile der Innenkanüle, wie bspw. eine Betätigungshandhabe, durch stark wirkende Zugkräfte zerstörerisch entfernt.

Der Zugriff von Daumen und Zeigefinger erfolgt somit im Bereich der Ausnehmungen (3), d.h. in den Eingriffmulden, an dem Rand (41) der Innenkanüle (4) sehr ergonomisch, so dass die Zugkräfte von Daumen und Zeigefinder optimal auf die Innenkanüle (4) übertragen werden können, um diese aus der Außenkanüle (1) durch Herausziehen zu entfernen.

Die Außenkanüle (1) und die Innenkanüle (4) bestehen aus Kunststoff, wie PVC, Silikon oder thermoplastischen Polyurethan, oder aus Metall, wie Sterlingsilber.

Die Außenkanüle (1) und die Innenkanüle (4) können gemäß dem Stand der Technik als Standardbogen von 80° bis 110° ausgeführt sein.

Alle in der Beschreibung und den nachfolgenden Ansprüchen dargestellten Merkmale können sowohl einzeln, als auch in beliebiger Kombination miteinander, erfindungswesentlich sein.

### Bezugszeichenliste

- 1: - Außenkanüle
- 11: - proximaler Teil
- 12: - distaler Teil
- 2: - Kanülenschild
- 3: - Ausnehmungen
- 4: - Innenkanüle
- 41: - wulstartiger Rand

## Patentansprüche

1. Anordnung in Form einer Tracheostoma- Prothese zum leichten Einsetzen und leichten Entnehmen einer Innenkanüle bestehend aus einer schlauchförmigen Außenkanüle (1) mit einem proximalen Teil (11) sowie einem distalen Teil (12) und einer schlauchförmigen Innenkanüle (4), wobei die Innenkanüle (4) in die Öffnung am proximalen Teil (11) der Außenkanüle (1) einführbar und durch Rast- oder Haltemittel arretierbar ist, und der äußere Rand des proximalen Teils (11) der Außenkanüle (1) zwei sich gegenüberliegende Ausnehmungen (3) aufweist, **dadurch gekennzeichnet, dass** die Ausnehmungen (3) kreisbogenförmig, für Daumen und Zeigefinger eingreifbar ausgestaltet sind und Innenkanüle an ihrem proximalen Ende einen wulstartigen Rand (41) aufweist.

2. Anordnung gemäß Anspruch 1, **dadurch gekennzeichnet, dass** die Außenkanüle (1) am proximalen Teil (11) mit einem Kanülenschild (2) versehen ist.

3. Anordnung gemäß Anspruch 1, **dadurch gekennzeichnet, dass** die Außenkanüle (1) und die Innenkanüle (4) aus Kunststoff, wie PVC, Silikon oder thermoplastischen Polyurethan, oder aus Metall, wie Sterlingsilber, bestehen.

4. Anordnung gemäß Anspruch 1, **dadurch gekennzeichnet, dass** die Außenkanüle (1) und die Innenkanüle (4) als Standardbogen von 80° bis 110° ausgeführt sind.

## Claims

1. An arrangement in the form of a tracheostoma prosthesis for the easy insertion and easy removal of an inner cannula, consisting of a tubular outer cannula (1) with a proximal part (11) and a distal part (12) and a tubular inner cannula (4), wherein the inner cannula (4) can be guided into the opening at the proximal part(11) of the outer cannula (1) and can be locked by locking or retaining means, and the outer edge of the proximal part (11) of the outer cannula (1) is provided with two recesses (3), arranged opposite to each other, **characterized in that** the recesses (3) have the shape of a circular arc and are designed to be grasped in by thumb and index finger, and the inner cannula (4) has a bead-like edge (41) at its proximal end.

2. The arrangement according to claim 1, **characterized in that** the outer cannula (1) is provided with a cannula shield (2) at the proximal part (11).

3. The arrangement according to claim 1, **characterized in that** the outer cannula (1) and the inner cannula (4) are made of plastic material, such as PVC, silicone or thermoplastic polyurethane, or of metal, e.g. sterling silver.

4. The arrangement according to claim 1, **characterized in that** the outer cannula (1) and the inner cannula (4) are designed as a standard arc from 80° to 110°.

## Revendications

1. L'invention concerne un système se présentant sous la forme d'une prothèse de trachéostome et destiné à faciliter l'introduction et le retrait d'une canule interne et se composant d'une canule externe (1) tubulaire, comportant une partie proximale (11) et une partie distale (12), et d'une canule interne (4) tubulaire, cette dernière pouvant être introduite dans l'ouverture sur la partie proximale (11) de la canule externe (1) et pouvant être bloquée par l'intermédiaire d'un moyen de verrouillage ou de maintien, et le bord extérieur de la partie proximale (11) de la canule extérieure (1) présentant deux évidements (3) opposés, conçus en arc de cercle, permettant l'introduction du pouce et de l'index, et la canule interne présentant, à son extrémité proximale, une bordure (41) en forme de bourrelet.

2. Dispositif suivant la revendication 1 est **caractérisé en ce que** la canule externe (1) est munie d'un écran d'aiguille (2) sur la partie proximale (11).

3. Dispositif suivant la revendication 1 est **caractérisé en ce que** la canule externe (1) et la canule interne (4) sont constituées d'un matière plastique comme PVC, silicone ou polyuréthane thermoplastique ou d'un métal comme l'argent sterling.

4. Dispositif suivant la revendication 1 est **caractérisé en ce que** la canule externe (1) et la canule interne (4) sont réalisées comme coude standard de 80° à 110°.
